## Europäisches Patentamt

### European Patent Office

### Office européen des brevets

(11) Publication number: **0 292 445 B1**

(12) **EUROPEAN PATENT SPECIFICATION**

(45) Date of publication of the patent specification: **26.09.90**

(21) Application number: **88830214.8**

(22) Date of filing: **17.05.88**

(51) Int. Cl.⁵: **A61M 1/18, B01D 63/04**

(54) Hollow-fibre oxygenation device.

(30) Priority: **18.05.87 IT 6743187**

(43) Date of publication of application:
**23.11.88 Bulletin 88/47**

(45) Publication of the grant of the patent:
**26.09.90 Bulletin 90/39**

(84) Designated Contracting States:
**DE ES FR GB**

(56) References cited:
**EP-A- 0 103 899**
**EP-A- 0 114 732**
**US-A- 4 368 118**

(73) Proprietor: **SORIN BIOMEDICA S.p.A., Strada per Crescentino, I-13040 Saluggia (Vercelli)(IT)**

(72) Inventor: **Conti, Mauro, Via Augero, 8/A, I-10038 Verolengo Torino(IT)**
Inventor: **Donato, Mario, Via Crescentino, 28, I-13040 Saluggia Vercelli(IT)**
Inventor: **Zantonelli, Piero, Via Gravere, 20, I-10145 Torino(IT)**

(74) Representative: **Bosotti, Luciano et al, c/o Jacobacci-Casetta & Perani S.p.A. Via Alfieri, 17, I-10121 Torino(IT)**

## Description

The present invention relates to blood-oxygenation devices and concerns, in particular, a device having the features set forth in the pre-characterising portion of Claim 1.

Oxygenation devices of this type, which are known, for instance, from EP-A 0 114 732 are preferably designed for use in a surgical environment for establishing blood circulation outside the body to maintain physiological levels of oxygen and carbon dioxide in the blood, are well known in the art.

The gas-exchange between the two phases, blood/oxygen-carrying flow, takes place through the semipermeable walls of the fibres and leads to enrichment of the blood with oxygen and the simultaneous removal of carbon dioxide therefrom.

Usually, in the devices in question, which are intended to be kept vertical during use, the blood flow to be oxygenated enters the device at the bottom and is discharged, after the oxygen exchange, from the top.

At least two different solutions are known in the art for the production of oxygenation devices, of the type specified above, in which the blood passes within the fibres.

According to the first solution, the device is constituted essentially by a tubular casing which surrounds the bundle of fibres and has two end parts forming respective manifolds, or inlet and outlet chambers, for the blood. Two generally discoidal sealing masses formed at the opposite ends of the bundle of fibres separate the two blood-collection chambers from the central cavity of the device itself. The flow of oxygen-carrying gas is circulated in the latter cavity, usually in the opposite direction (countercurrent) to the blood flow.

In the other solution (which is that which will be referred to in the detailed description of the invention below), the device has a tubular core which extends along the central axis of the fibre bundle. The wall of the core has holes through which the oxygen-carrying gas emerges, diffusing through the bundle of fibres in a generally radially direction thereof.

In both solutions, which provide for the passage of the blood within the fibres, a problem may arise due to the presence of air bubbles which have entered the blood-flow inadvertently, upstream of the oxgenation device or through the semipermeable walls of the fibres. There is a risk of these bubbles being drawn, with the oxygenated blood, into the patient's body, where they could result in the formation of embolisms or other dangerous ocurrences.

In order to avoid this problem, it is usual to provide an element which acts as a trap for the oxygen bubbles in series in the blood line which connects the output of the oxygenation device with the patient's body. See, in that respect, also EP-A 0 114 732, page 11, lines 1 to 3.

The object of the present invention is to provide a blood-oxygenation device in which the function of trapping or capturing any bubbles present in the blood is carried out, at least in part, within the oxygenator, making the extracorporeal circulation system simpler and less exacting, both structurally and in terms of bulk.

According to the present invention, this object is achieved by virtue of a blood-oxygenation device of the type specified above, having the further characteristics called for in Claim 1.

The collecting chamber preferably has a portion which is lowermost in use, the connector being situated in this lowermost portion.

The blood-collecting chamber usually has a shape which diverges overall towards a region of maximum vertical extent in its position of use, so that this region has an uppermost part in which the bubbles collect and a lowermost part in which the blood discharge connector is situated.

A vent duct for the bubbles may, to advantage, be provided in this uppermost region.

The invention will now be described purely by way of non-limiting example, with reference to the appended drawing which shows the end part of a hollow-fibre oxygenation device in axial section. This relates, specifically, to the end which is intended to be at the top in use.

According to a widely-known solution, the oxygenation device is constituted by a tubular casing 1 provided with a central core 2, which is also tubular, so as to define a space of circular-annular section in which a bundle of semi-permeable fibres 3 is disposed.

A blood flow passes through the cavities within the fibres 3, and, in the arrangement illustrated in the drawing, flows from the bottom upwards.

The wall of the central core 2 has a plurality of holes 4 through which a flow of oxygen-carrying gas, which reaches the interior of the central cavity 5 of the core 2 (by known means), can diffuse radially into the space containing the fibres 3 so as to flow over the surfaces of these fibres.

Gas exchange thus takes place between the blood and the oxygen-carrying flow through the semipermeable walls of the fibres 3 causing enrichment of the blood with oxygen and simultaneous removal of the carbon dioxide in the blood.

The device thus carries out a function wholly analogous to that usually fulfilled by the lungs. For this reason, devices of the type described are sometimes also known as "artificial lungs".

The upper ends of the fibres 3 are embedded in a sealing mass 6 of a material such as polyurethane or the like, but with the ends of the fibres left open.

Usually (also according to a widely-known solution) the ends of the fibres 3 are encased in the sealing mass 6 and then the resulting complex is cut. This removes the end portions of the fibres which may be blocked by the sealing material, enabling the blood which is circulated within the fibres 3 to flow through them freely. The main function of the sealing mass 6 is to separate the intermediate region of the device, indicated 7, into which the oxygen-carrying gas diffuses from the central core 2, from two end chambers which constitute manifolds for collecting the blood to be oxygenated and the oxygenated blood.

The drawing shows only the end chamber 8 which collects the oxygenated blood discharged vertically from the fibres 3.

The chamber 8 is defined by a shaped end part 9 of the casing 1. The part 9 is generally known in the art as the "cap".

The end part 9 has at least one connector 10 through which the oxygenated blood which collects in the chamber 8 can be returned to the patient's body.

The chamber 8 is defined by the internal surface of the cap 9 which is generally annular in shape as well as by the end plate 11 which faces the end openings of the fibres 3.

More precisely, the chamber 8 can be seen to include:

a radially inner portion 12 of small, constant height (measured axially of the device, that is in the direction in which the fibres 3 extend) (of the order of a mm), and

a radially outer portion 13 of greater height, ideally surrounding the edge of the end plane 11 of the bundle of fibres 3, which is bounded by a deflector element 14.

The heights of both the peripheral portion 13 of the chamber 8 defined by the cap 9 and of the deflector element 14 which is fixed to the end edge of the central part of the casing 1, (still measured axially of the device) increase gradually towards the zone in which the connector 10 is situated.

More particularly, in the zone furthest from the connector 10, the height of the deflector element 14 corresponds approximately to the height of the inner portion 12 of the chamber 8. The height of the deflector element reaches its maximum in the region in which the connector 10 is situated. The height of chamber 8 reaches its maximum extent, axially of the device, in this same region.

In this region, the chamber 8 therefore has an uppermost part 15 and a lowermost part 16. The connector 10 is located in this lowermost part, which is generally below the end plane 11 of the bundle of fibres.

An aperture 17 is, on the other hand, provided in the uppermost part 15 of the chamber 8 in the wall of the cap 9, and a vent device 18 can be inserted therein.

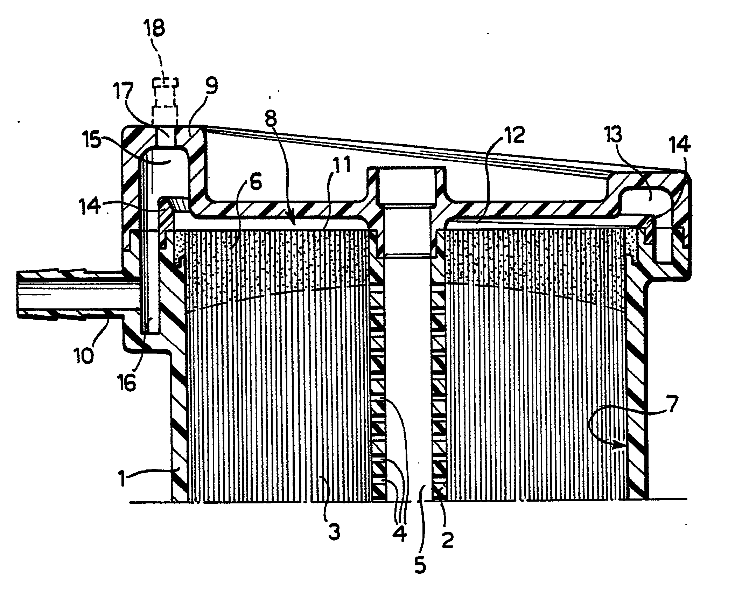

In operation, the oxygenation device according to the invention is kept in the vertical position, that is, in a position which corresponds, in practice, to that illustrated in Figure 1.

The oxygenated blood emerges vertically from the fibres 3 and collects in the radially-inner portion 12 of the chamber 8.

Moving generally radially of the core 2, the oxygenated blood then spreads out towards the radially-outer region 13 of the chamber 8. In this movement, the blood encounters the deflector element 14 which deflects the flow upwards.

The blood which collects in the region 13, around the entire periphery of the chamber 8, tends to flow towards the outlet connector 10, that is towards the peripheral region of the cap 9 in which the uppermost part 15 and the lowermost part 16, and hence the connector 10, are situated.

This movement is facilitated by the fact that the chamber 8 (or, more precisely, its radially-outer portion 13) has dimensions which increase gradually towards the region in which the uppermost part 15 and the lowermost part 16 are situated.

The result of this shape of the chamber 9 is that any air bubbles present in the oxygenated blood tend to collect towards the uppermost part 15, whilst the blood flows towards the lowermost part 16 where the connector 10 is situated.

Thus, the uppermost part 15 acts as a trap for any bubbles in the oxygenated blood.

The bubbles which collect in the uppermost part 15 may be removed through the vent device 18 connected to the aperture 17.

The device 18 may, for example, be connected through a blood-flow line to the reserve and filtration unit (cardiotome) which is normally connected to the oxygenation device. This unit (not illustrated in the drawing), collects blood in the operating area through one or more intake lines and filters from it any fragments of extraneous material or air bubbles. The blood, thus purified, can then be recycled to the inlet of the oxygenator. The line which connects the vent device 18 to the cardiotome is thus a line which partially recycles the blood output by the oxygenator. The line may always be open and, in this case, a small proportion of the blood is recycled taking with it any bubbles which have collected, or may be opened only intermittently when there is a need to eliminate a bubble.

## Claims

1. A blood-oxygenation device comprising a bundle of hollow fibres (3) for carrying a blood flow within them and intended to be flowed over by an oxygen-carrying flow (4), said bundle having an end plane (11) intended to face upwardly in use, and a casing (1) for containing the fibres (3), having a shaped end part (9) which together with said end plane (11) of the bundle defines a collecting chamber (8) for the oxygenated blood which is open to the presence of bubbles, and has at least one blood-discharge connector (10), characterised in that the blood discharge-connector (10) is situated in a position generally below said end plane (11) of the bundle, whereby the blood-discharge connector itself (10) is located in a position generally out of alignment with the path of propagation of the bubbles within the collecting chamber (8).

2. A device according to Claim 1, characterised in that the collecting chamber (8) has a part (16) which is lowermost in use and in that the connector (10) is located in this lowermost part (16).

3. A device according to Claim 1 or Claim 2, characterised in that the collecting chamber (8) has a shape which diverges overall towards a region (15, 16) of maximum vertical extent, in its position of use, so that the region of maximum vertical extent has an uppermost part (15) and a lowermost part (16) and in that the connector (10) is located in the lowermost part.

4. A device according to Claim 3, characterised in that it includes a vent duct for the bubbles (17, 18) located in the uppermost part (15).

5. A device according to one of Claims 1 to 4, characterised in that the bundle of fibres (3) has an end plane (11) delimiting the blood collecting chamber (8) and intended to face upwardly, in use and in that the end plane (11) is surrounded by a deflector element (14) which projects relative to the end plane (11).

6. A device according to Claim 5, characterised in that the projection of the deflector element (14) from the end plane (11) increases towards the blood discharge connector (10).

7. A device according to any one of the preceding claims, characterised in that the blood collecting chamber (8) is generally annular in shape with a radially inner portion (12) of substantially constant height, and a radially outer portion (13) whose height increases gradually towards the blood discharge connector (10).

## Patentansprüche

1. Vorrichtung zur Blut-Oxygenation mit einem Bündel von Hohlfasern (3), die von einer Blutströmung durchflossen und von einer Sauerstoffträger-Strömung (4) überströmt werden können, wobei dieses Bündel eine im Betrieb nach oben weisende Stirnfläche (11) aufweist, und einem Gehäuse (1) zur Aufnahme der Fasern (3), das einen ausgeformten Endbereich (9) besitzt, der zusammen mit der Stirnfläche (11) des Bündels eine Sammelkammer (8) für das mit Sauerstoff angereicherte Blut begrenzt und für vorhandene Blasen offen ist und das wenigstens ein Verbinderteil (10) für die Blutentnahme besitzt, dadurch gekennzeichnet, daß das Verbinderteil (10) für die Blutentnahme in einer Position unterhalb der genannten Stirnfläche (11) des Bündels liegt, so daß das Verbinderteil (10) für die Blutentnahme sich in einer Stellung befindet, die nicht mit dem Ausbreitungsweg der Blasen in der Sammelkammer (8) fluchtet.

2. Vorrichtung nach Anspruch 1, dadurch gekennzeichnet, daß die Sammelkammer (8) einen Teil (16) aufweist, der während des Betriebs zuunterst liegt, und daß das Verbinderteil (10) in diesem zuunterst liegenden Teil (16) angeordnet ist.

3. Vorrichtung nach Anspruch 1 oder 2, dadurch gekennzeichnet, daß die Sammelkammer (8) eine Form hat, die in Gebrauchsstellung insgesamt in Richtung auf einen Bereich (15, 16) maximaler vertikaler Ausdehnung divergiert, so daß der Bereich maximaler vertikaler Ausdehnung einen zuoberst liegenden Teil (15) und einen zuunterst liegenden Teil (16) besitzt, und daß das Verbinderteil (10) in dem zuunterst liegenden Teil angeordnet ist.

4. Vorrichtung nach Anspruch 3, dadurch gekennzeichnet, das eine Entlüftungsleitung (17, 18) für die Blasen vorgesehen ist, die in dem zuoberst liegenden Teil (15) angeordnet ist.

5. Vorrichtung nach einem der Ansprüche 1 bis 4, dadurch gekennzeichnet, daß das Bündel von Fasern (3) eine Stirnfläche (11) besitzt, die die Blutsammelkammer (8) begrenzt und im Betrieb nach oben weist, und daß die Stirnfläche (11) von einem Deflektorelement (14) umgeben ist, das relativ zu der Stirnfläche (11) vorsteht.

6. Vorrichtung nach Anspruch 5, dadurch gekennzeichnet, daß der über die Stirnfläche (11) vorstehende Teil des Deflektorelements (14) in Richtung auf das Verbinderteil (11) zur Blutentnahme größer wird.

7. Vorrichtung nach einem der vorhergehenden Ansprüche, dadurch gekennzeichnet, daß die Blut-Sammelkammer (8) im wesentlichen ringförmig ausgebildet ist und einen radial inneren Teil (12) mit im wesentlichen konstanter Höhe aufweist, sowie einen radial äußeren Teil (13), dessen Höhe in Richtung auf das Verbinderteil (10) zur Blutentnahme allmählich anwächst.

## Revendications

1. Dispositif d'oxygénation sanguine comportant un faisceau de fibres creuses (3) pour transporter à l'intérieur des fibres un flux sanguin et conçu pour être inondé par un flux transportant de l'oxygène (4) ledit faisceau présentant un plan d'extrémité (11) prévu pour être tourné vers le haut lors de son utilisation, et un logement (1) pour contenir les fibres (3), ayant une partie d'extrémité formée (9) qui, avec ledit plan d'extrémité (11) du faisceau, définit une chambre d'accumulation (8) pour le sang oxygéné qui est ouverte à la présence de bulles, et comporte au moins un connecteur d'évacuation du sang (10), caractérisé en ce que le connecteur d'évacuation du sang (10) est placé en une position généralement située en dessous dudit plan d'extrémité (11) du faisceau, le connecteur d'évacuation du sang (10) luimême étant de ce fait généralement situé hors d'alignement avec le trajet de propagation des bulles à l'intérieur de la chambre d'accumulation (8).

2. Dispositif selon la revendication 1, caractérisé en ce que la chambre d'accumulation (8) comporte une partie (16) qui est la plus basse lors de l'utilisation et en ce que le connecteur (10) est situé dans cette partie la plus basse (16).

3. Dispositif selon la revendication 1 ou la revendication 2, caractérisé en ce que la chambre d'accumulation (8) a une forme divergeant généralement vers une zone (15, 16) d'extension verticale maximum, dans sa position d'utilisation, de sorte que la région d'extension verticale maximum a une partie la plus élevée (15) et une partie la plus basse (16) et que le connecteur (10) est situé dans la partie la plus inférieure.

4. Dispositif selon la revendication 3, caractérisé en ce qu'il comprend un conduit de ventilation pour les bulles (17, 18) situé dans la partie la plus élevée (15).

5. Dispositif selon l'une des revendications 1 à 4, caractérisé en ce que le faisceau de fibres (3) a un plan d'extrémité (11) délimitant la chambre d'accumulation du sang (8) et est conçu pour être tourné vers le haut, lors de l'utilisation, et en ce que le plan d'extrémité (11) est entouré par un élément déflecteur (14) qui forme une avancée par rapport au plan d'extrémité (11).

6. Dispositif selon la revendication 5, caractérisé en ce que l'avancée de l'élément déflecteur (14) depuis le plan d'extrémité (11) augmente en direction du connecteur d'évacuation du sang (10).

7. Dispositif selon l'une quelconque des revendications précédentes, caractérisé en ce que la chambre d'accumulation du sang (8) est généralement de forme annulaire avec une partie radialement intérieure (12) de hauteur globalement constante, et une partie radialement extérieure (13) dont la hauteur augmente graduellement en direction du connecteur d'évacuation du sang (10).